(19) Europäisches Patentamt
European Patent Office
Office européen des brevets

(11) Publication number: 0 268 331 B1

# (12) EUROPEAN PATENT SPECIFICATION

(45) Date of publication of patent specification: 08.07.92

(51) Int. Cl.[5]: C12P 7/22, //(C12P7/22, C12R1:40)

(21) Application number: 87202183.7

(22) Date of filing: 09.11.87

## (54) Microbial preparation of catechols.

(30) Priority: 20.11.86 GB 8627711

(43) Date of publication of application:
25.05.88 Bulletin 88/21

(45) Publication of the grant of the patent:
08.07.92 Bulletin 92/28

(84) Designated Contracting States:
AT BE CH DE ES FR GB GR IT LI LU NL SE

(56) References cited:
EP-A- 0 076 606
EP-A- 0 252 567

BIOCHEMISTRY, vol. 7, no. 7, July 1968, pages 2653-2662; D.T. GIBSON et al.: "Oxidative degradation of aromatic hydrocarbons by microorganisms. I. Enzymatic formation of catechol from benzene"

BIOCHEMISTRY, vol. 7, no. 11, November 1968, pages 3795-3802; D.T. GIBSON et al.: "Oxidative degradation of aromatic hydrocarbons by microorganisms. II. Metabolism of halogenated aromatic hydrocarbons"

(73) Proprietor: SHELL INTERNATIONALE RESEARCH MAATSCHAPPIJ B.V.
Carel van Bylandtlaan 30
NL-2596 HR Den Haag(NL)

(72) Inventor: Geary, Philip John
5 Old School Court
Egerton Ashford Kent(GB)
Inventor: Hawes, John Ernest
17 Woodstock Road
Sittingbourne Kent(GB)

EP 0 268 331 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a microbial process for the preparation of catechols.

The ability of certain organisms such as Pseudomonas putida to metabolise benzene and certain further degradation products is known from the work of Gibson et al, Biochemistry, 7(7), 1968, p. 2653; and Biochemistry, 9(7), 1970, p. 1631. Thus, the metabolism is believed to follow the following enzyme catalysed reaction sequence:

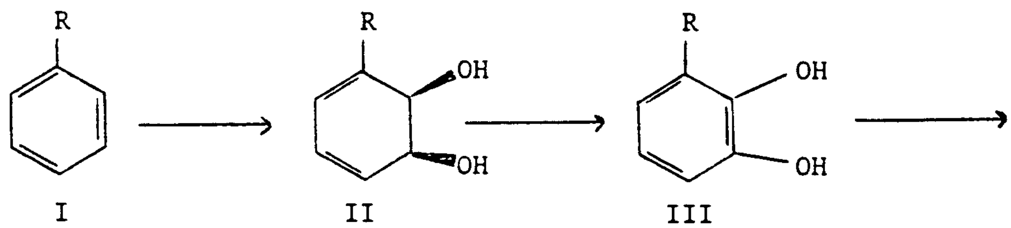

In accordance with this metabolic pathway, benzene (I; R = H) is converted by a dioxygenase to cis-1,2-dihydroxy-cyclohexa-3,5-diene (II; R = H) (sometimes known as "cis-benzene glycol" or "benzene dihydrodiol") which under the action of a diol dehydrogenase is converted to catechol (III; R = H) which is enzymatically converted to further degradation products. A related pathway, where R is methyl, is believed to occur for toluene metabolism using Pseudomonas putida (Gibson et al, Biochemistry, 9(7), 1970, p. 1627).

The microbial preparation of compounds of formula (II) containing a cis-1,2-dihydroxy-cyclohexa-3,5-diene ring has been described by Taylor (U.S. Patent No. 4,508,822) using mutant strains of P.putida. Gibson et al, Biochemistry, 12(8), 1973, p. 1520 have shown that cell free extracts of P.putida grown on ethylbenzene can be used to obtain a catechol product from a compound of formula (II) where R is ethyl.

Catechols would be useful products to obtain by a biochemical process. However it has been found difficult to control the enzyme catalysed reaction sequence from (I) to (III) to obtain compounds of formula (III) in sufficient yield to give a commercially viable process.

According to the present invention, we provide a microbial process for the preparation of a catechol of formula (IV)

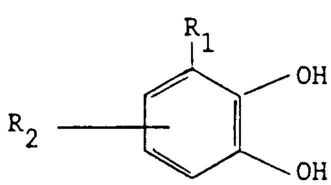

(IV)

where $R_1$ and $R_2$ are each selected from hydrogen, halogen, cyano, -COOH (and derivatives thereof such as unsubstituted or substituted amides) alkyl, aryl and cycloalkyl (which alkyl, aryl and cycloalkyl groups may be substituted), comprising supplying a compound of formula (V)

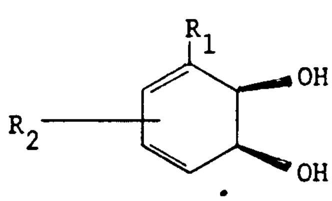

(V)

where $R_1$ and $R_2$ are as defined above, to a culture in a suitable medium of a mutant strain of a benzene-metabolising microorganism, which mutant strain contains a cis-benzene glycol dehydrogenenase and is capable of accumulating the required catechol of formula (IV) and subsequently recovering a compound of formula (IV) therefrom.

Preferably, although not necessarily, the compound of formula (V) has also been obtained by a microbial process from a compound of formula (VI)

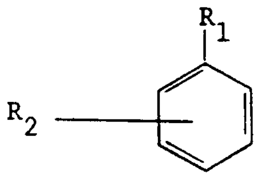

(VI)

where $R_1$ and $R_2$ are as defined above, by supplying the compound of formula (VI) to a culture in a suitable medium of a mutant strain of a benzene-metabolising microorganism which mutant strain contains a dioxygenase and is capable of accumulating the required cis-1,2-dihydroxy-cyclohexa-3,5-diene of formula (V). The compound of formula (V) may be directly employed after removal of the microorganism, but without product isolation, in the process of the invention for conversion to the catechol (IV). Alternatively the compound of formula (V) may be recovered before it is supplied to the mutant strain containing cis-benzene glycol dehydrogenase.

In this specification, unless indicated otherwise, any alkyl group preferably contains from 1 to 6 carbon atoms; any cycloalkyl group preferably contains from 3 to 6 carbon atoms; and any aryl group is preferably a phenyl group.

$R_2$ in the formulae (IV), (V) and (VI) given above is preferably hydrogen. $R_1$ is preferably selected from hydrogen, halogen, cyano, alkyl and $CX_3$ (where X is a halogen, preferably fluorine). $R_1$ is preferably fluorine, the resulting product being 3-fluorocatechol. 3-Fluorocatechol is difficult to prepare by purely chemical means and is consequently expensive. It is a useful intermediate in the production of, for example, pharmaceuticals and organo-fluorine agrochemicals.

The presently preferred benzene-metabolising microorganism from which suitable mutant strains may be obtained for conversion of (V) to (IV), and also for microbial preparation of (V) from (VI), is P.putida. A preferred wild type microorganism is that deposited with effect from 6th December 1985 with the National Collection of Industrial Bacteria, Torry Research Station, Aberdeen, Scotland and assigned the numerical designation NCIB 12190 and referred to herein as "P.putida NCIB 12190". Other suitable microorganisms include those described in U.S. patent No. 4,508,822, namely Pseudomonas putida NCIB 11767 and Pseudomonas putida NCIB 11680.

The mutant strain for conversion of (V) to (IV) and containing cis-benzene glycol dehydrogenase is preferably obtained by a selection procedure which comprises mutating a wild type strain of Pseudomonas putida by chemical or physical means, allowing the mutated bacteria to grow in the presence of a carbon source, exposing the grown bacteria to benzene or a substituted benzene and selecting those mutant strains of Pseudomonas putida which have accumulated catechol or its substituted analogues. Likewise the mutant strain for conversion of (VI) to (V) and containing a dioxygenase has preferably been obtained by a selection procedure which comprises mutating a wild type strain of P.putida by chemical or physical means, allowing the mutated bacteria to grow in the presence of a carbon source, exposing the grown bacteria to benzene or a substituted benzene and selecting those mutant strains of P.putida which have accumulated cis-dihydroxycyclohexadiene or its substituted analogues. If necessary a re-selection process may be carried out to obtain a suitable culture for use in the conversion of (VI) to (V) or of (V) to (IV).

It has been found advantageous in preparing suitable organisms for the conversion reactions to grow the organisms in a suitable growth medium followed by a "reactivation" step involving further growth with no additional nutrient, for example for from 1 to 2 hours. It has also been found advantageous to dilute the culture medium by the addition of an equal volume of a solution of the substrate when converting (V) to (IV).

The mutations may be carried out by chemical means, employing, for example, as mutating agent N-methyl-N′-nitro-N-nitrosoguanidine (referred to hereinafter as "NTG"). Other mutating agents may be employed, such as 2-aminopurine or 5-bromouracil. The mutation may also be carried out by physical means for example by ultraviolet irradiation or using other ionizing radiation methods.

Examples of preferred carbon sources are succinic acid, fumaric acid, and gluconic acid, suitably in the form of salts such as disodium succinate, disodium fumarate, and sodium gluconate, fructose and glycerol. Other suitable carbon sources include sucrose, galactose, lactose and citrates.

The nutrient medium for growth may be any suitable for the growth of the mutant strains, an example being a minimal salts medium containing sodium succinate.

Preferred mutant strains for the conversion of (V) to (IV) are those obtained by ultraviolet irradiation and designated UV-mutants A, B, C and D, described hereinafter. A particularly preferred mutant strains for the conversion of (VI) to (V) is obtained by NTG-mutation and designated NTG mutant F herein.

A preferred medium for the conversion of (V) to (IV), or for the conversion of (VI) to (V), comprises

sodium succinate as carbon source, for example in combination with ammonium sulphate and yeast extract. Other suitable carbon sources include fumarates, gluconates, fructose, glycerol, sucrose, galactose, lactose and citrates.

During the conversion of (V) to (IV), the culture medium is preferably maintained at not more than 5% air saturation, more preferably at not more than 2% air saturation. The temperature is conveniently in the range of from 15 to 35 ° C, preferably from 25 to 30 ° C. The pH of the medium is preferably in the range of from 6.5 to 7.5, more preferably from 6.8 to 7.2. The concentration of cells in the medium is relatively critical, and is preferably in the range of from 0.5 to 2 grammes dry weight/litre. We have found that when the concentration of cells is high (eg. 2 g/l and above), some over oxidation may occur.

The desired product compound may be recovered from the resulting fermentation broth by any suitable means such as absorption onto granulated charcoal or solvent extraction.

We have found that by using a cis-1,2-dihydroxy-cyclohexa-3,5-diene compound of formula (V) as substrate for the mutant strain containing cis-benzene glycol dehydrogenase, good yields of product catechol can be obtained. This is in contrast to the poor yield obtainable by using directly as substrate a compound of formula (VI). Thus we have found that, for example, when using fluorobenzene as substrate for a mutant strain such as that designated UV-mutant A, optimum concentrations of product 3-fluorocatechol of only about 0.6 g/l can be obtained. This is in spite of the fact that, in theory, UV-mutant A contains both the necessary enzymes to convert fluorobenzene to 3-fluorocatechol, i.e. the dioxygenase for conversion of fluorobenzene to 3-fluoro-cis-1,2-dihydroxycyclohexa-3,5-diene as well as the diol dehydrogenase for conversion to 3-fluorocatechol.

Surprisingly, on the other hand, when carrying out the process in stepwise manner in accordance with the present invention, so that 3-fluoro-cis-1,2-dihydroxy-cyclohexa-3,5-diene, rather than fluorobenzene, is used as substrate for UV-mutant A, concentrations of product 3-fluorocatechol of more than 3 g/l can be obtained.

Although not wishing to be bound by such a theory, it is believed that the catechols, e.g. 3-fluorocatechol, may inhibit the action of the dioxygenase required to convert (VI) to (V) while not inhibiting the diol dehydrogenase required to convert (V) to (IV). This was not to be expected and could not be predicted from the initial experiments on the metabolic pathway carried out by Gibson et al as referenced above.

The invention will now be further described by way of example.

Growth Media

The compositions of two of the growth media used in the following examples are given below in Table 1.

ASM - minimal salts medium
NFSM - nitrogen-free salts medium

**Table 1**

| | amounts per litre | |
|---|---|---|
| | ASM | NFSM |
| $Na_2HPO_4$ | 0.866g | 7g |
| $K H_2PO_4$ | 0.531g | 3g |
| $NH_4Cl$ | 0.535g | - |
| $K_2 SO_4$ | 0.174g | 0.174g |
| $MgSO_4.7H_2O$ | 0.037g | 0.037g |
| $CaCl_2.2H_2O$ | 0.00735g | 0.00735g |
| TK3 (T/E) (see below) | 1.0 ml | 1.0 ml |
| $FeSO_4.7H_2O$(0.1M) | 0.2 ml | 0.2 ml |

pH 6.8

Compositions of TK/3 Trace Element Solution:

This contained per litre the following components: $ZnSO_4.7H_2O$ (0.288g); $MnSO_4.4H_2O$ (0.224g); $H_3BO_3$ (0.0618g); $CuSO_4.5H_2O$ (0.1248g); $Na_2MoO_4.2H_2O$ (0.0484g); $CoCl_2.6H_2O$ (0.0476g); KI (0.083g); 1M $H_2SO_4$ (1 ml).
Further growth media used were:

| | | |
|---|---|---|
| dYT medium | ----- | 16 g Bacto tryptone |
| | | 10 g Bacto yeast extract |
| | | 5 g NaCl/l |
| Yeast extract medium (YEM) | ----- | 10 g/l disodum succinate $.6H_2O$ |
| | | 2 g/l $(NH_4)_2SO_4$ |
| | | 3 g/l yeast extract (Difco) |
| | | 0.4 g/l $MgSO_4.7H_2O$ |

0.04 g/l Bacto-peptone in
25 mM potassium phosphate buffer,
final pH 7.0.

Colorimetric determination of cis-dihydroxycyclohexadienes and catechols

The method of Friestad et al., Analytical Chemistry 41, 1969, pp. 1750 - 1754 was used. Solutions to be tested for catechols were mixed with an equal volume of 3-methyl-2-benzothiazolinone hydrazone hydrochloride (0.05% w/v in water) and an equal volume of ceric ammonium sulphate (0.2% w/v in 0.4% w/v sulphuric acid). Optionally, a borate-NaOH-EDTA buffer was added after a few minutes, as described by Friestad et al. The intensities of the colours which developed were compared visually or, alternatively, were measured spectrophotometrically at 520 nm. Cis-dihydroxycyclohexadienes do not produce colours under these conditions. Therefore a second sample of each test solution was acidified with sulphuric or hydrochloric acid before the colour test, to convert the dihydroxycyclohexadienes quantitatively to phenols. The difference in colour intensity given by the acidified and unacidified samples is indicative of the content of dihydroxycyclohexadienes.

Gas Chromatography (GC)

Aqueous samples (0.5μl) were chromatographed on a Hewlett Packard 25-metre high capacity flexible silica capillary column coated with 5%-phenylmethylsilicone, using helium as carrier gas. The column was held at 130°C and eluted compounds were detected by a flame ionization detector.

Thin Layer Chromatography (TLC)

Aqueous samples (5 μl) were run on Merck Kieselgel 50 F254 plates developed with 90:10:1 (v/v) n-propanol-water-formic acid. Dihydroxycyclohexadiene content was visualised under short wave UV light and catechol was detected by spraying with 2,6-dichloroquinone-4-chloroimide (2% in ethanol).

High Pressure Liquid Chromatography (HPLC)

This was carried out using a 15 cm Spherisorb S85 μcolumn with a solvent of 20:80 v/v methanol:water plus 0.1% v/v acetic acid pumped at 0.1 ml/min. Detection was at 220 nm using a Cecil CE 212

spectrophotometer.

Example 1 - The preparation of UV mutant of P.putida containing diol dehydrogenase.

Aliquots (1 ml) of a suspension of P.putida NCIB 12190 in phosphate buffer pH7 were spread onto nutrient agar plates. The plates were irradiated using a Chromatolux u.v. lamp for 5 to 30 minutes. The plates were incubated at 30°C overnight and then placed in an atmosphere of fluorobenzene at 30°C and incubated for a further 24 hours. 34 surviving colonies were purified and tested for their ability to accumulate fluorocatechol in shake flask experiments. Fluorocatechol was assayed by gas chromatography. In comparative experiments, one mutant strain, hereinafter referred to as UV-mutant A, accumulated 0.68 g/l fluorocatechol in 3-4 hr, and a second mutant strain, designated UV-mutant B, accumulated 0.56 g/l in 3-4 hr. Under the same conditions the wild strain NCIB 12190 accumulated 0.41 g/l. Two further strains, accumulating 0.5 to 0.6 g/l and 0.62 g/l were also isolated and designated UV-mutant C and UV-mutant D.

Example 2 - The preparation of NTG mutants of P.putida containing dioxygenase.

Pseudomonas putida NCIB 12190 was grown overnight at 30°C in dYT medium. The culture was subcultured 1/20 into 10 ml fresh dYT and incubated for a further 4 hours. The bacteria were harvested by centrifugation, washed in NFSM and again harvested by centrifugation before resuspension in NFSM (3 ml) and adjustment of the OD 600 nm to 3.0.

A 1 ml aliquot in an "Eppendorf" tube was incubated at 30°C for 15 mins and then NTG (N-methyl-N'-nitro-N-nitrosoguanidine-5mg/ml in dimethyl suphoxide) added to give 50 µg/ml. The tube contents were briefly mixed and incubated at 30°C for 15 mins. Mutation was stopped by chilling in ice, centrifugation and washing (3 times in saline-0.85% NaCl). The bacteria were diluted in saline and 0.1 ml aliquots plated out onto 100 ASM plates each including 0.05% sodium succinate. The plates were incubated at 30°C for 48 hours.

After incubation, small "micro" colonies were visible on the plates, the level of succcinate having been only sufficient for a small amount of growth. The plates were then exposed to benzene vapour for 24 h at 30°C resulting in a wide variety of colony sizes visible on the plates. In an initial selection procedure, small colonies on the plates (which are small because of their inability to grow on benzene) (20-30/plate) were picked using sterile cocktail sticks into 96 well microtitre plates (Titertek) containing 50 µl ASM + 0.5% sodium succinate per well. The plates were incubated overnight at 30°C in a sealed box to prevent evaporation and the microtitre plates replicated onto square (120 mm) plates of commercial nutrient agar (Oxoid, to provide controls) using a 96 prong replicating device. The agar plates were incubated overnight and the microtitre plates were exposed to benzene vapour for 3 hours. Subsequent assay was by the colorimetric method described above. Those mutants developing the most intense colour were selected for further testing for the accumulation of cis-dihydroxycyclohexadiene and identification of the best mutants for production. The selected best mutants were tested using shake flask cultures and the colorimetric determination again carried out. Controls were run using a culture of wild type P.putida NCIB 12190 to which chloramphenicol had been added as a protein synthesis inhibitor, although no chloramphenicol was added to the mutant strains. Confirmatory HPLC analyses of some of the supernatants were carried out.

From each of six independent starting cultures of P.putida NCIB 12190 mutated with NTG, 100 plates were inoculated giving approximately 100,000 colonies, 13,500 of which were picked into microtitre plates and 435 initially selected for colour testing. 90 of these were further investigated after colour testing and TLC analysis and shake flask experiments carried out on 14 of the mutants.

The cultures were grown overnight in ASM plus 0.5% sodium succinate, harvested by centrifugation, washed and resuspended in NFSM. The cultures were exposed to benzene and the supernatants assayed for catechol or catechol plus cis-dihydroxycyclohexadiene. For control runs, wild type P.putida NCIB 12190 was employed with chloramphenicol (1 mg/ml) added to the NFSM medium.

One of those mutants which was unable to produce catechol but which accumulated cis-dihydroxycyclohexadiene was selected and designated NTG-mutant F. The results of this mutant are shown in Table 2.

Table 2

| OD 520 nm | | | |
|---|---|---|---|
| Catechol | | Catechol plus cis-dihydroxycyclohexadiene | |
| after 2½ hrs | after 7 hrs | after 2½ hrs | after 7 hrs |
| 0.04 | 0.06 | 0.27 | 0.54 |

Example 3 - The use of NTG mutant F in the production of 3-fluoro-cis-1,2-dihydroxycyclohexa-3,5-diene using succinate as carbon source.

8 Litres of YEM were inoculated in a stirred fermenter with a 20 hour shake flask culture of NTG Mutant F grown on the same medium (50 ml). The organism was grown at aerating conditions of 500 rpm and 500 ml. air/min. for 19 hours with a continuous feed of concentrated nutrient (320 g of disodium succinate and 64 g of ammonium sulphate in 1 litre of 0.025 M potassium phosphate buffer pH 7.2) at 40 ml/hour. Oxygen transfer was increased by increasing the stirrer speed to 550 rpm and the aeration rate to 700 ml air/min for 30 min and then set to conditions of 500 rpm; 600 ml air/min; equilibrium oxygen tension 30% air saturated initially).

Fluorobenzene was metered by a pump (Gilson Model 302) at 50 μl/min (100-125 mg/l equilibrium concentration in the reaction). Prior to reaction, the optical density was determined as 3.11 corresponding to a dry cell weight of 3.9 g/l. Production of 3-fluoro-cis-1,2-dihydroxycylohexa-3,5-diene (II; R = F) was monitored by gas chromatography and the concentration reached 2.8 g/l after 24 hr.

The product was absorbed on granulated charcoal and recovered from the charcoal by extraction in a Soxhlet apparatus with a mixture of diethyl ether and methanol (4:l v/v). Evaporation of the solvent left a solid which was recrystallised from a mixture of diethyl ether and pentane to give colourless needles, m.p. 73-74°C (decomp.).

UV Spectrum ($H_2O$): λ max 259 nm (ε 3150).
Circular dichroism) ($H_2O$: λ max 255 nm (Δε - 1.9).
Mass spectrum m/z 130 (15%,$M^+$), 112 (65%), 84 (100%).
$^1$H-NMR spectrum ($CDCl_3$): θ 5.88 (1H, mult, J = 10, 6.5, 6, 2 Hz; 5-C-H), 5.71 (1H, dd, J = 10, 3 Hz; 6-C-H), 5.60 (1H, dd, J = 11, 6.5 Hz; 4-C-H), 4.51 (1H, br.mult, J = 6, 3, 2 Hz; 1-C-H), 4.29 (1H, tr, J = 6, 6 Hz; 2-C-H), 2.40 (2H, br.s, O-H) p.p.m.

Example 4 - Conversion of 3-fluoro-cis-1,2-dihydroxy cyclohexa-3,5-diene to 3-fluorocatechol using different UV-mutants

The various UV-mutants A, B, C and D prepared as described in Example 1 were grown in shake flasks for 17 hours in a medium comprising sodium succinate heptahydrate (10g/l), yeast extract (3 g/l), ammonium sulphate (2 g/l), magnesium sulphate hexahydrate (0.4 g/l) and Bactopeptone (0.04 g/l), together with trace metals to a cell concentration of approximately 1.5g/l dry cell weight. To each flask of 250 ml capacity and containing 50 ml culture was added a sample of 3-fluoro-cis-1,2-dihydroxycyclohexa-3,5-diene, previously isolated and purified, and equal to a concentration of 4 g/l. The flasks were incubated in the shake mode at about 30°C, the oxygen requirements being met by ingress of air via the porous closure. The results are given in Table 3 below.

Table 3

| Diol dehydrogenase | UV-Mutant A | UV-Mutant B | UV-mutant C | UV-mutant D |
|---|---|---|---|---|
| Initial reaction rate (g/l/hr) after 15 minutes | 1.0 | 2.03 | 1.71 | 1.96 |
| % Diol converted after 7 hours | 76 | 96 | 93 | 90.5 |
| concentration of 3-fluorocatechol in g/l after 7 hours | 0.61 | 0.88 | 1.12 | 1.78 |
| % Diol converted after 24 hours | 92.7 | 97.7 | 98.4 | 100 |

It is to be noted that the concentration of 3-fluorocatechol can be increased by working at sufficient dilution with controlled aeration.

Example 5 - Conversion of 3-fluoro-cis-1,2-dihydroxy-cyclohexa-3,5-diene to 3-fluorocatechol

UV-mutant A, prepared as described in Example 1, was grown on Yeast Extract Medium to a cell concentration of 2g/l dry weight. The culture was then diluted 1:1 with molasses medium comprising 25mM potassium phosphate buffer pH 7.2 containing 2g/l ammonium sulphate; 18.75g/l cane molasses; 3g/l yeast extract, 0.4g/l magnesium sulphate and 0.04g/l bactopeptone, and crystalline 3-fluoro-cis-1,2-dihydroxycyclohexa-3,5-diene (3.5g/l) added. The flasks containing the culture were then incubated in Shake mode at about 30°C, the oxygen requirements being met by ingress air via a porous flask closure. After 8 hrs, more than 80% of the diol had been converted and the concentration of 3-fluorocatechol in the medium was 3.1g/l.

Comparative Example - The use of UV mutant A in the production of 3-fluorocatechol

8 Litres of YEM in a fermenter were inoculated with 30 ml of a 16 hour shake culture of UV-mutant A grown at 30°C on the same medium. The organism was grown under conditions of oxygen limitation (200 rpm, 350 ml air/min) for 17 hours, during which time a zero reading was recorded on the oxygen electrode. After 17 hours the aeration rate was increased to 400 rpm; 450 ml air/min to stimulate growth and maintained for 1 hour to provide an actively growing culture of dry weight 0.6 g/l (viable cell count 1.9 $\times 10^9$/ml). Fluorobenzene (10 ml) was added and aeration decreased to 320 rpm; 150 ml air/min. At intervals, controlled by a sensor (katherometer) in the off gas, the air was saturated with fluorobenzene by passage through a bubbler containing that compound, maintaining an equilibrium concentration of fluorobenzene of about 300 mg/l in the reaction mixture. 3-Fluorocatechol production was monitored by GLC and estimated to have reached 0.45 g/l after 6 hours and 0.62 g/l after 24 hours. The pH was. controlled throughout at 7.2 by automatic addition of $H_2SO_4$.

Characterisation of Pseudomonas Putida NCIB 12190

Pseudomonas putida NCIB 12190 has been characterised and identified by the NCIB as follows:
Tests were at 25°C and growth was on LAB M Nutrient Agar unless otherwise stated.

Cell Morphology

After growth for 24 hours at 30°C on succinate agar, transferred to Nutrient broth + 0.75%w agar, by phase contrast at × 630 magnification cells are small short rods or cocci in clusters.

Gram negative

Spores -

Motility +

Colonial Morphology

After 48 hours growth, colonies are round, regular, entire, smooth, opaque, low convex, off-white and less than 1mm in diameter.

Growth on Glucose Peptone Water Sugars
37° +
41°C -

Catalase +

Oxidase, Kovacs +

O-F glucose Oxidative

"O-F glucose" was performed using the oxidation-fermentation medium of Hayward and Hodgkiss, J.Gen. Microbiol. 26 (1961) 133-140, supplemented with 1%w filter-sterilised D-glucose. A tube sample was inoculated and incubated for 14 days.

Pseudomonas putida NCIB 12190 can conveniently be stored on nutrient agar slopes at 4°C, or as a freeze-dried material.

**Claims**

**1.** A microbial process for the preparation of a catechol of formula (IV)

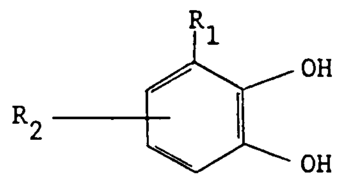

(IV)

where $R_1$ and $R_2$ are each selected from hydrogen, halogen, cyano, -COOH (and derivatives thereof), alkyl, aryl and cycloalkyl (which alkyl, aryl and cycloalkyl groups may be substituted), comprising supplying a compound of formula (V)

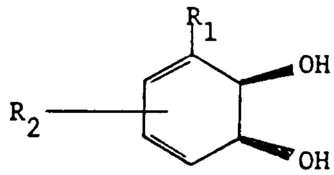

(V)

where $R_1$ and $R_2$ are as defined above, to a culture in a suitable medium of a mutant strain of a benzene-metabolising microorganism, which mutant strain contains a cis-benzene glycol dehydrogenenase and is capable of accumulating the required catechol of formula (IV) and subsequently recovering a compound of formula (IV) therefrom.

2. A process according to claim 1 wherein $R_2$ is hydrogen.

3. A process according to claim 1 or 2 wherein $R_1$ is fluorine.

4. A process according to claim 1, 2 or 3 wherein the mutant strain containing a cis-benzene glycol dehydrogenase has been obtained by mutating a wild type strain of Pseudomonas putida, preferably P.putida NCIB 12190.

5. A process according to claim 4, wherein the mutant strain has been obtained by ultraviolet irradiation.

6. A process according to any of the preceding claims wherein the process is carried out in a medium comprising succinic acid.

7. A process according to any one of the preceding claims wherein the compound of formula (V) has been obtained by a microbial process from a compound of formula (VI)

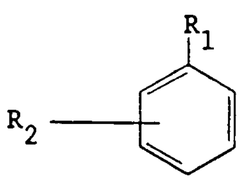

(VI)

where $R_1$ and $R_2$ are as defined in claim 1, by supplying the compound of formula (VI) to a culture in a suitable media of a mutant strain of a benzene-metabolising microorganism which mutant strain contains a dioxygenase and is capable of accumulating the required cis-1,2-dihydroxycyclohexa-3,5-diene of formula (V).

8. A process according to claim 7, wherein the compound of formula (V) is directly employed for supply to the mutant strain containing a cis-benzene glycol dehydrogenase without product isolation but after removal of the microorganism containing a dioxygenase.

9. A process according to claim 7 or 8 wherein the mutant strain containing a dioxygenase has been obtained by mutating a wild type strain of Pseudomonas putida, preferably P.putida NCIB 12190.

10. A process according to claim 9 wherein the mutant strain containing a dioxygenase has been obtained by chemical means using as mutating agent N-methyl-N′-nitro-N-nitrosoguanidine.

**Revendications**

1. Un procédé microbien pour la préparation d'un pyrocatéchol de formule (IV)

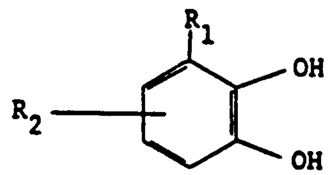

(IV)

où $R_1$ et $R_2$ sont choisis chacun parmi l'hydrogène, les halogènes, des groupes cyano, -COOH (et ses dérivés), alcoyle, aryle et cycloalcoyle (lesquels groupes alcoyle, aryle et cycloalcoyle peuvent être substitués), selon lequel on introduit un composé de formule (V)

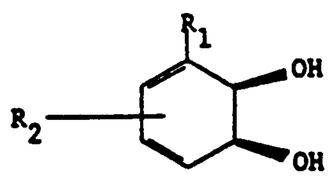

(V)

où $R_1$ et $R_2$ sont tels que définis ci-dessus dans une culture dans un milieu approprié d'une souche mutante d'un micro-organisme métabolisant le benzène, laquelle souche mutante contient une cis-benzène glycol déshydrogénase et est capable d'accumuler le pyrocatéchol désiré de formule (IV) et on recueille ensuite un composé de formule (IV).

**2.** Un procédé selon la revendication 1, dans lequel $R_2$ est de l'hydrogène.

**3.** Un procédé selon la revendication 1 ou 2, dans lequel $R_1$ est du fluor.

**4.** Un procédé selon la revendication 1, 2 ou 3, dans lequel la souche mutante contenant une cis-benzène glycol déshydrogénase a été obtenue par mutation d'une souche de type sauvage de Pseudomonas putida, de préférence P.putida NCIB 12190.

**5.** Un procédé selon la revendication 4, dans lequel la souche mutante a été obtenue par irradiation ultraviolette.

**6.** Un procédé selon l'une quelconque des revendications précédentes, qui est mis en oeuvre dans un milieu comprenant de l'acide succinique.

**7.** Un procédé selon l'une quelconque des revendications précédentes, dans lequel le composé de formule (V) a été obtenu par un procédé microbien à partir d'un composé de formule (VI)

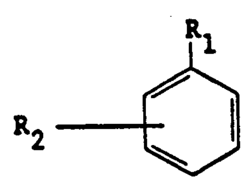

(VI)

où $R_1$ et $R_2$ sont tels que définis dans la revendication 1, en introduisant le composé de formule (VI) dans une culture dans un milieu approprié d'une souche mutante d'un micro-organisme métabolisant le benzène, laquelle souche mutante contient une dioxygénase et est capable d'accumuler le cis-1,2-dihydroxycyclohexa-3,5-diène désiré de formule (V).

**8.** Un procédé selon la revendication 7, dans lequel le composé de formule (V) est utilisé directement pour fourniture à la souche mutante contenant une cis-benzène glycol déshydrogénase sans isolement du produit, mais après élimination du micro-organisme contenant une dioxygénase.

**9.** Un procédé selon la revendication 7 ou 8, dans lequel la souche mutante contenant une dioxygénase a

été obtenue par mutation d'une souche de type sauvage de Pseudomonas putida, de préférence P.putida NCIB 12190.

**10.** Un procédé selon la revendication 9, dans lequel la souche mutante contenant une dioxygénase a été obtenue par des méthodes chimiques utilisant comme agent de mutation la N-méthyl-N'-nitro-N-nitroso-guanidine.

**Patentansprüche**

**1.** Mikrobielles Verfahren zur Herstellung eines Brenzcatechins der Formel (IV)

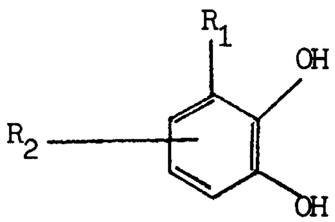

(IV),

worin $R_1$ und $R_2$ jeweils unter Wasserstoff, Halogen, Cyano, -COOH (und Derivaten hievon), Alkyl, Aryl und Cycloalkyl ausgewählt sind (wobei die Alkyl-, Aryl- und Cycloalkylgruppen substituiert sein können), umfassend das Einbringen einer Verbindung der Formel (V)

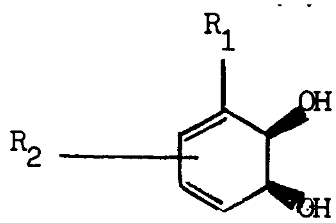

(V),

worin $R_1$ und $R_2$ wie vorstehend definiert sind, in eine Kultur eines mutanten Stammes eines Benzol-metabolisierenden Mikroorganismus in einem geeigneten Medium, welcher mutanter Stamm eine cis-Phenylglycoldehydrogenase enthält und zur Ansammlung des angestrebten Brenzcatechins der Formel (IV) befähigt ist, und ein anschließendes Gewinnen einer Verbindung der Formel (IV) hieraus.

**2.** Verfahren nach Anspruch 1, worin $R_2$ Wasserstoff bedeutet.

**3.** Verfahren nach Anspruch 1 oder 2, worin $R_1$ Fluor bedeutet.

**4.** Verfahren nach Anspruch 1, 2 oder 3, worin der eine cis-Phenylglycoldehydrogenase enthaltende mutante Stamm durch Mutieren eines Wildtypenstammes von Pseudomonas putida, vorzugsweise P. putida NCIB 12190, erhalten worden ist.

**5.** Verfahren nach Anspruch 4, worin der mutante Stamm durch Ultraviolettbestrahlung erhalten worden ist.

**6.** Verfahren nach einem der vorstehenden Ansprüche, worin das Verfahren in einem Bernsteinsäure enthaltenden Medium ausgeführt wird.

**7.** Verfahren nach einem der vorstehenden Ansprüche, worin die Verbindung der Formel (V) durch ein mikrobielles Verfahren aus einer Verbindung der Formel (VI)

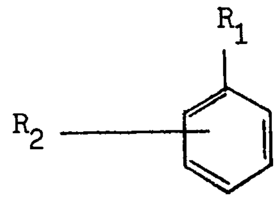

(VI),

worin $R_1$ und $R_2$ wie in Anspruch 1 definiert sind, durch Einbringen der Verbindung der Formel (VI) in eine Kultur eines mutanten Stammes eines Benzol-metabolisierenden Mikroorganismus in einem geeigneten Medium erhalten worden ist, welcher mutante Stamm eine Dioxygenase enthält und zur Ansammlung des angestrebten cis-1,2-Dihydroxycyclohexa-3,5-diens der Formel (V) befähigt ist.

**8.** Verfahren nach Anspruch 7, worin die Verbindung der Formel (V) direkt zur Speisung des eine cis-Phenylglycoldehydrogenase enthaltenden mutanten Stammes ohne Produktisolierung verwendet wird, aber nach Abtrennung des eine Dioxygenase enthaltenden Mikroorganismus.

**9.** Verfahren nach Anspruch 7 oder 8, worin der eine Dioxygenase enthaltende Stamm durch Mutieren eines Wildtypenstammes von Pseudomonas putida, vorzugsweise P. putida NCIB 12190, erhalten worden ist.

**10.** Verfahren nach Anspruch 9, worin der eine Dioxygenase enthaltende mutante Stamm auf chemischem Wege unter Verwendung von N-Methyl-N'-nitro-N-nitroso-guanidin als Mutationsmittel erhalten worden ist.